(19) Europäisches Patentamt / European Patent Office / Office européen des brevets

(11) EP 2 606 912 A1

# (12) EUROPEAN PATENT APPLICATION

(43) Date of publication:
**26.06.2013 Bulletin 2013/26**

(51) Int Cl.:
***A61K 49/04*** *(2006.01)*

(21) Application number: **12197686.4**

(22) Date of filing: **18.12.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **23.12.2011 KR 20110140954**
**14.12.2012 KR 20120146878**

(71) Applicants:
- **Central Medical Service Co., Ltd.**
  **Seoul 143-811 (KR)**
- **Kim, Boo Geun**
  **Junggye-dong, Nowon-gu**
  **Seoul**
  **139-923 (KR)**

(72) Inventors:
- **KIM, Boo-Geun**
  **139-923 SEOUL (KR)**
- **KIM, Young-Il**
  **137-779 SEOUL (KR)**

(74) Representative: **Marconnet, Sébastien**
**Cabinet Marconnet**
**95bis rue Manin**
**75019 Paris (FR)**

## (54) Contrast medium composition with contrast enhancement effect by comprising highly concentrated agent

(57) The present invention relates to a contrast medium composition (formulation) comprising a highly concentrated contrast agent for use in an imaging method. The contrast medium composition comprises an aqueous contrast agent selected from the group consisting of iopentol, iotrolan, iohexol, ioversol, ioxilan, iodixanol and iobitridol in an amount of 360 mgI/mL to 450 mgI/mL.

The contrast medium composition according to the present invention, which comprises iodine working as a contrast agent in a higher concentration than the concentration used before, shows higher contrast enhancement effect in both of arterial phase and portal phase when used for liver CT than a composition comprising the contrast medium in a concentration used before. Accordingly, images having excellent resolution and discrimination can be obtained.

Fig. 5

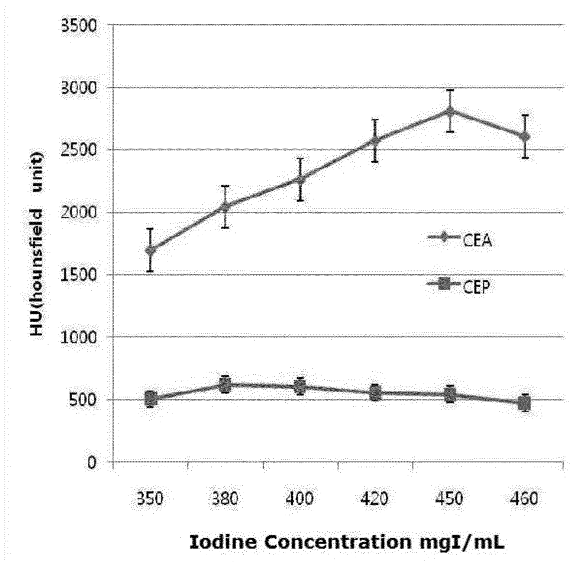




Printed by Jouve, 75001 PARIS (FR)

## Description

### Field of the Invention

The present invention relates to a contrast medium composition for using in an imaging method, particularly to a contrast medium composition comprising a highly concentrated contrast agent so as to have high contrast enhancement effect.

### Background of the Invention

CT (computed tomography) is an imaging method that X-ray is radiated from various directions to the desired human body part, the transmitted X-ray is collected using a detector, and then a computer calculates the X-ray absorption difference of the part followed by reconstructing the data using mathmatical technique. The basic principle of CT is that an X-ray tube radiates X-ray beam while rotating around a section of the human body; a detector collects strength data; and then a computer calculates absorption strength by part using the data and reconstructing images using the calculated data followed by showing them on a monitor.

CT has better resolution and contrast in distinguishing blood, cerebrospinal fluid, white matter, gray matter, tumor and the like than previous X-ray images, and can express absorption difference at a fine part. Accordingly, it occupies a very important position in image diagnosis fields.

The most significant feature of CT is using X-ray, but the x-ray examination can't distinguish a structure having similar density. The X-ray of short wavelength is effective for imaging a dense structure such as bones, but it has low sharpness for imaging soft tissues. Accordingly, there is a limit to distinguish tissues and their peripheral areas with only X-ray examination. Therefore, contrast media are needed to artificially provide contrast between the examined organs and their peripheral tissues. Namely, unlike gastrointestinal radiologic diagnosis, the X-ray examinations such as urography and angiography are needed to administer safe, aqueous and radiopaque contrast media intravenously.

Presently, iodine-based contrast media are being most widely used, and specifically, in triiodobenzene-based X-ray contrast medium fields, non-ionic triiodobenzoic acid derivatives such as iopamidol, iohexol and ioversol were developed and rapidly grown:

Iomeprol {$C_{17}H_{22}I_3N_3O_8$; N,N'-bis(2,3-dihydroxypropyl)-5-[(hydroxyacetyl)methylamino]-2,4,6-triiodo-1 ,3-benzene dicarboxamide; CAS [RN] [78649-41-9]},

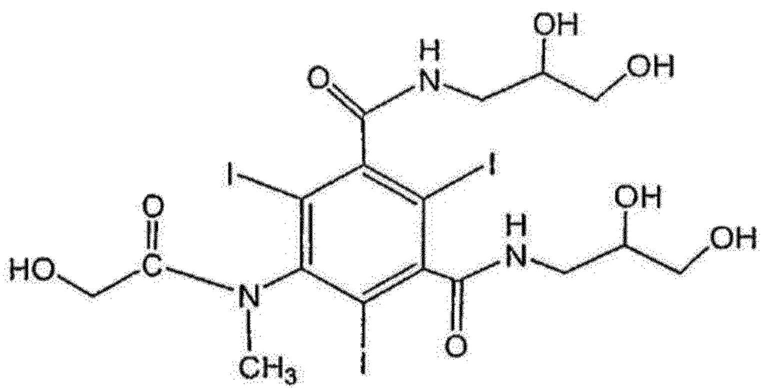

Iopromide {$C_{18}H_{24}I_3N_3O_8$; N,N'-bis(2,3-dihydroxypropyl)-2,4,6-triiodo-5-[(methoxyacetyl)amino]-N-meth yl-1,3-benzene dicarboxamide; CAS [RN] [73334-07-3]},

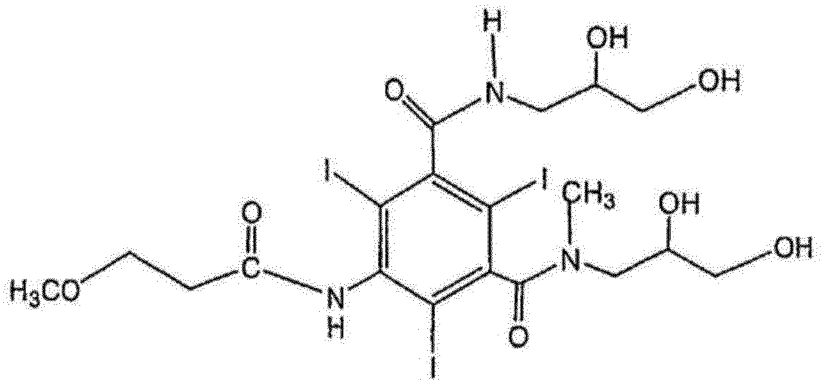

Ioversol {$C_{18}H_{24}I_3N_3O_9$; N,N'-bis(2,3-dihydroxypropyl)-5-[(hydroxyacetyl)(2-hydroxyethyl)amino]-2,4, 6-triiodo-1,3-benzenedicarboxamide; CAS [RN][87771-40-2]},

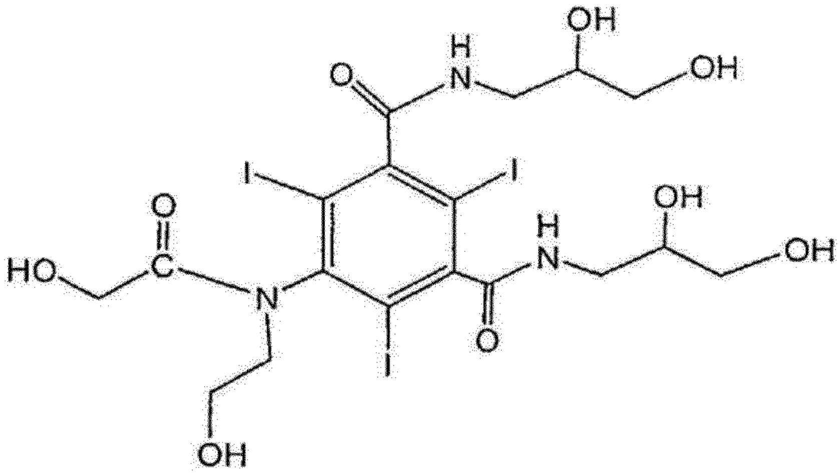

Iohexol {$C_{19}H_{26}I_3N_3O_9$; 5-[acetyl(2,3-dihydroxypropyl)amino]-N,N'-bis(2,3-dihydroxypropyl)-2,4,6-triiodo-1,3-benzene dicarboxamide; CAS [RN] [66108-95-0]},

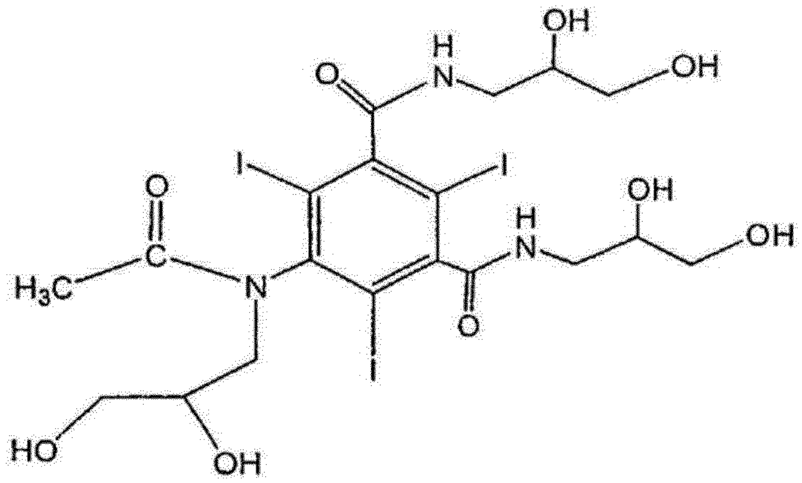

Iopentol {$C_{20}H_{28}I_3N_3O_9$; 5-[acetyl(2-hydroxy-3-methoxypropyl)amino]-N,N'-bis(2,3-dihydroxypropyl)-2 ,4,6-triiodo-1,3-benzene dicarboxamide; CAS [RN] [89797-00-2]},

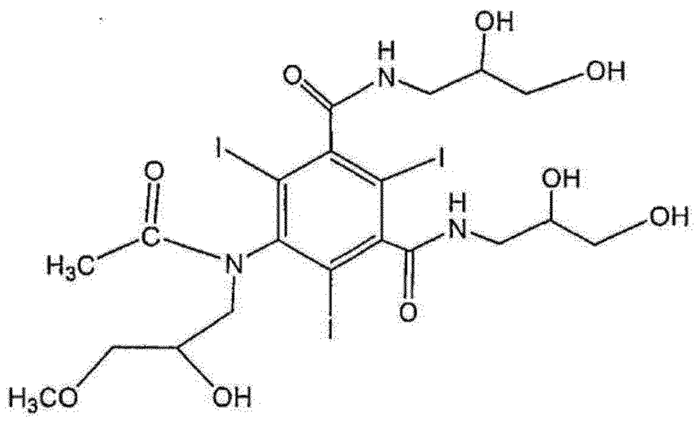

Iopamidol {$C_{17}H_{22}I_3N_3O_8$; 5-[(2-hydroxy-1-oxopropyl)amino]-N,N'-bis(2-hydroxy-1-(hydroxymethyl)ethy 1)-2,4,6-triiodo-1,3-benzene dicarboxamide; CAS [RN][60166-93-0]},

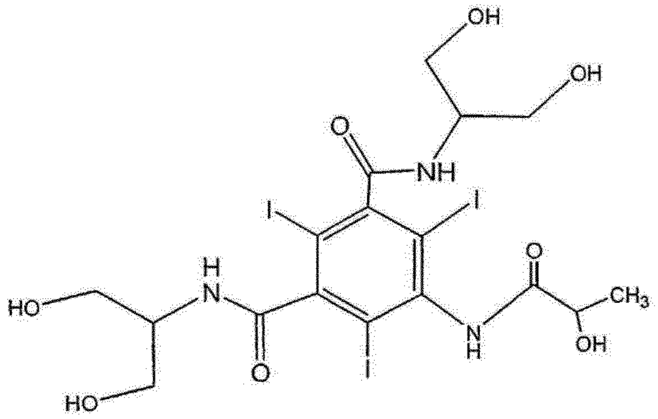

Iobitridol {$C_{20}H_{28}I_3N_3O_9$; N,N'-bis(2,3-dihydroxypropyl)-5-[[3-hydroxy-2-(hydroxymethyl)-1-oxopropyl] amino]-2,4,6-triiodo-N,N'-dimethyl-1,3-benzene dicarboxamide; CAS [RN][136949-58-1]}.

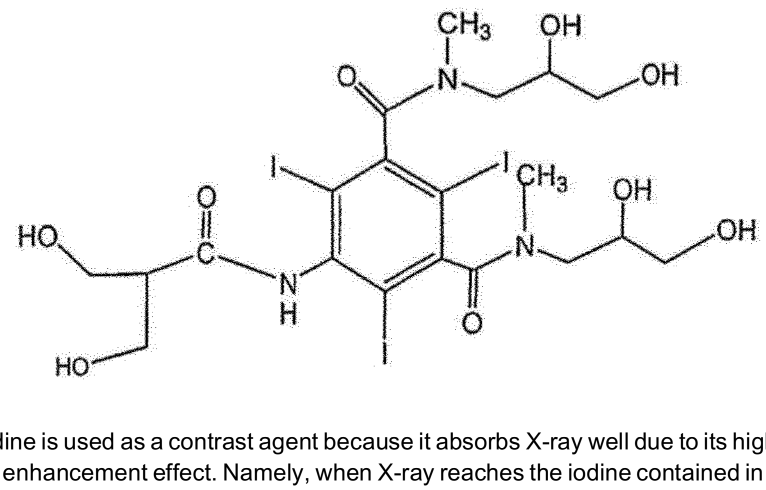

Like this, the iodine is used as a contrast agent because it absorbs X-ray well due to its high density and thereby shows excellent contrast enhancement effect. Namely, when X-ray reaches the iodine contained in the contrast medium, X-ray is absorbed and looks white on CT. Accordingly, the organ to diagnose looks bright.
Injection speed and iodine concentration of the contrast medium may vary according to a purpose of the test. Particularly, the iohexol was previously clinically admitted to be used for enhancing CT contrast in amounts of 140 mgI/mL, 180 mgI/mL, 240 mgI/mL, 300 mgI/mL, and up to 350 mgI/mL.
However, due to explosive demands and expectations (early diagnosis and precise diagnosis) of medical market for the development of medical skills and diagnosis, the contrast medium composition, which was previously admitted,

could not satisfy the demands for more precise and accurate diagnosis of image diagnosis. Therefore, in order to achieve high quality imaging, a novel contrast medium composition comprising a highly concentrated contrast agent and thereby showing contrast enhancement effect is needed.

## Summary of the Invention

In order to solve the above-described problems associated with prior art, the present invention is objected to provide a novel contrast medium composition having a highly concentrated contrast agent and/or low Hounsfield value.

In order to accomplish one object of the present invention, the present invention provides a contrast medium composition comprising an aqueous contrast agent selected from the group consisting of iopentol,_iotrolan, iohexol, ioversol, ioxilan, iodixanol and iobitridol in an amount of 360 mgI/mL to 450 mgI/mL.

In the present invention, the contrast medium composition comprising the aqueous contrast agent in a concentration of 360 mgI/mL to 450 mgI/mL refers a composition containing iodine atoms of 360 mg to 450 mg per the contrast medium composition of 1 ml.

The present invention is characterized that the aqueous contrast agent is iohexol. The iohexol is known for having generally lower chemical toxicity and neurotoxicity than other non-ionic contrast agents because it contains six intramolecular -OH groups so as to show high hydrophilicity. The iopromide can't be used for imaging spiral cord due to its high neurotoxicity, and the ioversol doesn't have any indications for imaging abdominal cavity and spiral cord. But the iohexol has very wide adaptive domain and, for this reason, the iohexol is the best-selling contrast agent in the world.

The present invention is characterized that the concentration of the iohexol as the aqueous contrast agent in the contrast medium composition is higher as 360 mgI/mL to 450 mgI/mL, preferably 380 mgI/mL, 400 mgI/mL or 420 mgI/mL than the concentration used before as 350 mgI/mL. In the present invention, when the concentration of the iohexol in the contrast medium composition is 360 mgI/mL to 450 mgI/mL, the contrast medium composition can provide contrast enhancement effect in both of arterial phase and portal phase when used for liver CT imaging.

The present invention is characterized that contrast medium composition further comprises an aqueous buffer, sterile water for injection, a chelating agent and pH controlling agent.

The present invention is characterized that the aqueous buffer is tromethamine. In the present invention, the aqueous buffer comprises 2-amino-2-(hydroxymethyl)-1,3-propanediol, also known as the tromethamine. The tromethamine is toxicologically acceptable, and is described in U. S. Pharmacopoeia, XXI edition, U. S. Pharmacopeial Convention, Rockville, MD. 1985 (U.S.P XXI) at page 1102. The tromethamine (Chemical Name: 2-amino-2-hydroxymethyl-1,3-propanediol) is also known as other names of Trimethylol aminomethane; Tris(hydroxymethyl)aminomethane; Trisamine; Tris buffer; Trometamol; Tromethane; THAM; TRIS; Talatrol; Trisamino; Tris-steril; and Trizma, as described in Merck Index(11th edition, Merck Co. Inc., Rahway, NJ. 1989). The tromethamine and its salts are known for working as a buffer in the pH range of 6-9.

The present invention is characterized that the chelating agent is at least one selected from the group consisting of ethylenediamine, edetate calcium disodium and edetate disodium.

The chelating agent added to the composition may any pharmaceutically acceptable chelating agent, but it may be ethylenediamine, edetate calcium disodium or edetate disodium, preferably. Among them, the edetate disodium is more preferable. The concentration of the chelating agent may be preferably 0.01 to 1 mg/mL, particularly 0.05 to 0.5 mg/mL. When the amount of the chelating agent is less than the range described above, stabilizing effect of the contrast medium comprising the iohexol and the like is not enough. When the amount of the chelating agent excesses the range described above, the stabilizing effect by the chelating agent reaches to the upper limit and, in addition, the excessive amount is not desirable regarding to the safety.

Regarding to stability of the contrast medium comprising the iohexol and the like, the pH of the contrast medium composition of the present invention can be preferably controlled to 7.0 to 8.0 by using pharmaceutically acceptable buffer for injection formulation such as hydrochloric acid or its salt, citric acid or its salt, phosphoric acid or its salt and acetic acid or its salt.

Further, the injection formulation of the present invention can be prepared by adding any necessary pharmaceutically acceptable isotonic agent, preservative and the like.

The present invention is characterized that CEA and CEP of the contrast medium composition comprising the aqueous contrast agent in a concentration of 360 mgI/mL to 450 mgI/mL satisfies the following relationship with $CEA_{350}$ and $CEP_{350}$ of the contrast medium composition comprising the aqueous contrast agent in a concentration of 350 mgI/ml.

$$CEA > CEA_{350}$$

$$CEP > CEP_{350}$$

The CEA is a contrast enhancement value in arterial phase, and it represents the result of measuring Hounsfield unit (HU) of the abdominal aorta at the level showing the celiac artery; and the CEP is a contrast enhancement value in portal phase, and it represents the result of measuring Hounsfield unit (HU) of the portal vein at the level showing the main portal vein.

Namely, the present invention shows an effect of improving the CEA and CEP in both of arterial phase and portal phase when used for liver CT imaging by using the iohexol at a higher concentration than the concentration of 350 mgI/mL, which was clinically used before.

The present invention is characterized that $CEA_{380}$ and $CEP_{380}$ of the contrast medium composition comprising the aqueous contrast agent in a concentration of 380 mgI/mL satisfies the following relationship with $CEA_{350}$ and $CEP_{350}$ when using the aqueous contrast agent in an amount of 350 mgI/ml.

$$1.7 \geq CEA_{380} / CEA_{350} \geq 1.1$$

$$1.7 \geq CEP_{380} / CEP_{350} \geq 1.1$$

The present invention is characterized that $CEA_{400}$ and $CEP_{400}$ of the contrast medium composition comprising the aqueous contrast agent in a concentration of 400 mgI/mL satisfies the following relationship with $CEA_{350}$ and $CEP_{350}$ when using the aqueous contrast agent in an amount of 350 mgI/ml.

$$1.7 \geq CEA_{400} / CEA_{350} \geq 1.1$$

$$1.7 \geq CEA_{400} / CEP_{350} \geq 1.1$$

The present invention is characterized that $CEA_{420}$ and $CEP_{420}$ of the contrast medium composition comprising the aqueous contrast agent in a concentration of 420 mgI/mL satisfies the following relationship with $CEA_{350}$ and $CEP_{350}$ when using the aqueous contrast agent in an amount of 350 mgI/ml.

$$1.7 \geq CEA_{420} / CEA_{350} \geq 1.1$$

$$1.7 \geq CEP_{420} / CEP_{350} \geq 1.05$$

In the present invention, the contrast medium composition is characterized by being generally used for liver CT imaging.

## Advantageous Effects of the Invention

The contrast medium composition according to the present invention, which comprises iodine working as a contrast agent in a higher concentration than the concentration used before, can provide CT images having excellent sharpness and discrimination due to its high contrast enhancement effect. Particularly, it shows contrast enhancement effect in both of arterial phase and portal phase when used for liver CT.

## Brief Description of Drawings

The above and other objects and features of the present invention will become apparent from the following description of the invention taken in conjunction with the following accompanying drawings, which respectively show:

FIG. 1: images of liver CT after administering a 380 mgI/ml iohexol injection formulation prepared by one Example of the present invention to a rabbit;

FIG. 2: images of liver CT after administering a 400 mgI/ml iohexol injection formulation prepared by one Example of the present invention to a rabbit;
FIG. 3: images of liver CT after administering a 420 mgI/ml iohexol injection formulation prepared by one Example of the present invention to a rabbit;
FIG. 4: images of liver CT after administering a 350 mgI/ml iohexol injection formulation prepared by one Comparative Example of the present invention to a rabbit; and
FIG. 5: a graph showing the changes of CEA and CEP values as the contrast enhancement effect according to the iodine amounts used in Examples of the present invention.

## Detailed Description of the Invention

The following Examples are intended to further illustrate the present invention by way of help understanding the present invention, and thus are not limitative of the present invention.

### Example 1: 380 mgI/ml Iohexol Injection Formulation

In order to make the iohexol concentration in a contrast medium composition to 380 mgI/ml, iohexol of 819.71 g, tromethamine of 1.31 g and edetate calcium disodium of 0.11 g were completely dissolved in water for injection (WFI) of 75 ml by mixing thereof at room temperature (RT). pH was controlled to 7.79 by using 1N hydrochloric acid solution, and WFI was added to make the final volume of the formulation 1 L. Then, the resulting solution was subjected to sterile filtration by using a micro filter. The obtained solution was filled to a 100 mL vial. Then, the vial was sealed with a rubber stopper and an aluminum cap to obtain an injection formulation.

### Example 2: 360 mgI/ml Iohexol Injection Formulation

An injection formulation was obtained by using a method similar with the method of Example 1 except for using iohexol of 776.62 g and tromethamine of 1.24 g in order to make the iohexol concentration to 360 mgI/ml.

### Example 3: 400 mgI/ml Iohexol Injection Formulation

An injection formulation was obtained by using a method similar with the method of Example 1 except for using iohexol of 862.80 g and tromethamine of 1.38 g in order to make the iohexol concentration to 400 mgI/ml.

### Example 4: 420 mgI/ml Iohexol Injection Formulation

An injection formulation was obtained by using a method similar with the method of Example 1 except for using iohexol of 905.88 g and tromethamine of 1.45 g in order to make the iohexol concentration to 420 mgI/ml.

### Example 5 : 450 mgI/ml Iohexol Injection Formulation

An injection formulation was obtained by using a method similar with the method of Example 1 except for using iohexol of 970.5 g and tromethamine of 1.55 g in order to make the iohexol concentration to 450 mgI/ml.

### Comparative Example 1: 350 mgI/ml Iohexol Injection Formulation

An injection formulation was obtained by using a method similar with the method of Example 1 except for using iohexol of 755.0 g and tromethamine of 1.21 g in order to make the iohexol concentration to 350 mgI/ml, which is previously admitted dosage of iohexol.

### Test Example 1: Measuring Viscosity, Osmosis and pH

Viscosity, osmosis and pH before and after controlling of the injection formulations of Examples 1 to 5 and Comparative Example 1 were measured, and the results thereof were listed in the following Table 1.

**Table 1**

| Classification | Iodine Concentration | Viscosity | Osmosis | pH |
|---|---|---|---|---|
| Example 1 | 380 mgI/ml | 19 | 905 | 7.79 |
| Example 2 | 360 mgI/ml | 10 | 810 | 7.75 |
| Example 3 | 400 mgI/ml | 37.5 | 1034 | 7.80 |
| Example 4 | 420 mgI/ml | 45.0 | 1096 | 7.79 |
| Example 5 | 450 mgI/ml | 71 | 1204 | 7.85 |
| Comparative Example 1 | 350 mgI/ml | 19 | 748 | 7.55 |

It was confirmed that viscosity and osmosis were increased with increased iodine concentration. pH was controlled to the range from 7.5 to 8.0.

**Test Example 2: imaging and Measuring HU (Hounsfield Unit) in Liver Tissue Using 380 mgI/ml iohexol injection formulation of Example 1**

The 380 mgI/ml iohexol injection formulation prepared in Example 1, was administered to each of seven rabbits, and liver CT images were taken. The resulting images were shown in FIG. 1.

Method for CT imaging: After anesthetizing the subject animal, a 22 G IV catheter was installed in the auricular vein of the rabbit followed by being connected to a power injector using a connector, and then a contrast medium (6.4 - 6.5 mL) was injected at the rate of 1.4 ml/sec followed by taking CT images with time difference using Definition (64 MDCT, Siemens, Germany). CT scan parameters were set follows: 100 mAs and 80 kVp table speed.

In the images in FIG. 1, the left image is an image in arterial phase, and the result of measuring Hounsfield unit (HU) of the abdominal aorta at the level showing the celiac artery. The measured Hounsfield unit (HU) was considered as the Hounsfield unit (HU) in arterial phase, i.e., $CEA_{380}$ (contrast enhancement value in arterial phase).

The right image in FIG. 1 is an image in portal phase, and the result of measuring Hounsfield unit (HU) of the portal vein at the level showing the main portal vein. The measured contrast enhancement value was considered as the Hounsfield unit (HU) in portal phase, i.e., $CEP_{380}$ (contrast enhancement value in portal phase), and the results were listed in the following Table 2 with $CEA_{380}$.

**Table 2**

| ID | $CEA_{380}$ | $CEP_{380}$ |
|---|---|---|
| 708 | 2157 | 531 |
| 709 | 1835 | 402 |
| 701 | 1358 | 538 |
| 702 | 1975 | 720 |
| 703 | 2323 | 633 |
| 704 | 2542 | 802 |
| 503 | 2115 | 691 |
| Average | 2043.6 | 616.7 |
| Standard Deviation | 379.2 | 135.7 |

**Test Example 3: Imaging and Measuring HU (Hounsfield Unit) in Liver Tissue Using 400 mgI/ml iohexol injection formulation of Example 3**

Using the 400 mgI/ml iohexol injection formulation prepared in Example 3, images were taken by the same method of Test Example 2 for three rabbits, and the resulting images were shown in FIG. 2. The results of measuring $CEA_{400}$ and $CEP_{400}$ of the images in FIG. 2 were listed in the following Table 3.

**Table 3**

| ID | $CEA_{400}$ | $CEP_{400}$ |
|---|---|---|
| 1005 | 2526 | 667 |
| 1006 | 2412 | 641 |
| 1007 | 1850 | 504 |
| Average | 2262.7 | 604.0 |
| Standard Deviation | 361.9 | 87.6 |

**Test Example 4: imaging and Measuring HU (Hounsfield Unit) in Liver Tissue Using 420 mgI/ml iohexol injection formulation of Example 4**

Using the 420 mgI/ml iohexol injection formulation prepared in Example 4, images were taken by the same method of Test Example 2, and the resulting images were shown in FIG. 3. The results of measuring $CEA_{420}$ and $CEP_{420}$ of the images in FIG. 3 were listed in the following Table 4.

**Table 4**

| ID | $CEA_{420}$ | $CEP_{420}$ |
|---|---|---|
| 1009 | 2574 | 552 |

**Test Example 5: imaging and Measuring HU (Hounsfield Unit) in Liver Tissue Using 350 mgI/ml iohexol injection formulation of Comparative Example**

Using the 350 mgI/ml iohexol injection formulation prepared in Comparative Example, images were taken by the same method of Test Example 2, and the resulting images were shown in FIG. 4. The results of measuring $CEA_{350}$ and $CEP_{350}$ of the images in FIG. 4 were listed in the following Table 5.

**Table 5**

| ID | $CEA_{350}$ | $CEP_{350}$ |
|---|---|---|
| 608 | 1971 | 493 |
| 610 | 1557 | 461 |
| 702 | 1872 | 553 |
| 703 | 1701 | 497 |
| 704 | 1521 | 623 |
| 705 | 1278 | 421 |
| 707 | 1959 | 473 |
| Average | 1694.1 | 503 |
| Standard Deviation | 258.3 | 66.4 |

CEA values and CEP values of Examples and Comparative Example measured in Test Example 2 to 5 were shown in FIG. 5 according to each iodine concentration.

As shown in FIG. 5, it was confirmed that CEA values were increased in the iodine concentration range of 360 to 450 mgI/ml and showed contrast enhancement effect in arterial phase, but CEP values were increased and then decreased and showed increasing and decreasing contrast enhancement effect in portal phase.

Further, it was confirmed that, when the iodine concentration was 460 mgI/ml or more, CEA values were decreased but CEP values were decreased below the iodine concentration of Comparative Example of 350 mgI/mL.

Accordingly, in the contrast medium composition according to the present invention, the iodine concentration may be in the range of 350 to 450 mgI/ml where showing contrast enhancement effect in arterial phase at liver CT imaging, and it may be preferably in the range of 380 to 420 mgI/ml, where showing contrast enhancement effect in both

arterial phase and portal phase.

Further, Hounsfield units (HU) in arterial phase and portal phase when conducting liver CT imaging in the iodine concentrations of 380, 400 and 420 mgI/ml and Hounsfield units (HU) in arterial phase and portal phase when conducting liver CT imaging in an iodine concentration of 350 mgI/ml were compared, and the results thereof were listed in the following Table 6.

**Table 6**

| | CEA / $CEA_{350}$ | CEP / $CEP_{350}$ |
|---|---|---|
| 380 mgI/ml | $CEA_{380}$ / $CEA_{350}$ = 1.206 | $CEP_{380}$ / $CEP_{350}$ = 1.226 |
| 400 mgI/ml | $CEA_{400}$ / $CEA_{350}$ = 1.335 | $CEP_{400}$ / $CEP_{350}$ = 1.201 |
| 420 mgI/ml | $CEA_{420}$ / $CEA_{350}$ = 1.519 | $CEP_{420}$ / $CEP_{350}$ = 1.097 |

As shown in Table 6, with increased iodine concentration than the concentration used before of 350 mgI/ml, Hounsfield unit (HU) ratio in arterial phase was increased and Hounsfield unit (HU) ratio in portal phase was decreased. When the iodine concentration was 380 to 420 mgI/ml, Hounsfield unit (HU) in both arterial phase and portal phase were higher than HU in an iodine concentration of 350 mgI/ml used before.

While the invention has been described with respect to the above specific embodiments, it should be recognized that various modifications and changes may be made and also fall within the scope of the invention as defined by the claims that follow.

## Claims

**1.** A contrast medium composition comprising an aqueous contrast agent selected from the group consisting of iopentol, iotrolan, iohexol, ioversol, ioxilan, iodixanol and iobitridol in an amount of 360 mgI/mL to 450 mgI/mL.

**2.** The contrast medium composition of claim 1, wherein the aqueous contrast agent is iohexol.

**3.** The contrast medium composition of claim 2, which comprises the aqueous contrast agent in a concentration of 380 mgI/mL to 420 mgI/mL.

**4.** The contrast medium composition of claim 2, which comprises the aqueous contrast agent in a concentration of 380 mgI/mL.

**5.** The contrast medium composition of claim 2, which comprises the aqueous contrast agent in a concentration of 400 mgI/mL.

**6.** The contrast medium composition of claim 2, which comprises the aqueous contrast agent in a concentration of 420 mgI/mL.

**7.** The contrast medium composition of claim 1, which further comprises aqueous buffer, sterile water for injection, chelating agent and pH controlling agent.

**8.** The contrast medium composition of claim 7, wherein the aqueous buffer is tromethamine.

**9.** The contrast medium composition of claim 7, wherein the chelating agent is at least one selected from the group consisting of ethylenediamine, edetate calcium disodium and edetate disodium.

**10.** The contrast medium composition of claim 1, whose pH is 7 to 8.

**11.** The contrast medium composition of claim 3, wherein CEA and CEP of the contrast medium composition comprising the aqueous contrast agent in a concentration of 360 mgI/mL to 450 mgI/mL satisfies the following relationship with $CEA_{350}$ and $CEP_{350}$ of the contrast medium composition comprising the aqueous contrast agent in a concentration of 350 mgI/ml.

$$CEA > CEA_{350}$$

$$CEP > CEP_{350}$$

**12.** The contrast medium composition of claim 4, wherein $CEA_{380}$ and $CEP_{380}$ of the contrast medium composition comprising the aqueous contrast agent in a concentration of 380 mgI/mL satisfies the following relationship with $CEA_{350}$ and $CEP_{350}$ of the contrast medium composition comprising the aqueous contrast agent in a concentration of 350 mgI/ml.

$$1.7 \geq CEA_{380} / CEA_{350} \geq 1.1$$

$$1.7 \geq CEP_{380} / CEP_{350} \geq 1.1$$

**13.** The contrast medium composition of claim 5, wherein $CEA_{400}$ and $CEP_{400}$ of the contrast medium composition comprising the aqueous contrast agent in a concentration of 400 mgI/mL satisfies the following relationship with $CEA_{350}$ and $CEP_{350}$ of the contrast medium composition comprising the aqueous contrast agent in a concentration of 350 mgI/ml.

$$1.7 \geq CEA_{400} / CEA_{350} \geq 1.1$$

$$1.7 \geq CEA_{400} / CEP_{350} \geq 1.1$$

**14.** The contrast medium composition of claim 6, wherein $CEA_{420}$ and $CEP_{420}$ of the contrast medium composition comprising the aqueous contrast agent in a concentration of 420 mgI/mL satisfies the following relationship with $CEA_{350}$ and $CEP_{350}$ of the contrast medium composition comprising the aqueous contrast agent in a concentration of 350 mgI/ml.

$$1.7 \geq CEA_{420} / CEA_{350} \geq 1.1$$

$$1.7 \geq CEP_{420} / CEP_{350} \geq 1.05$$

**15.** The contrast medium composition of claim 1, which is used for liver CT imaging.

Fig. 1

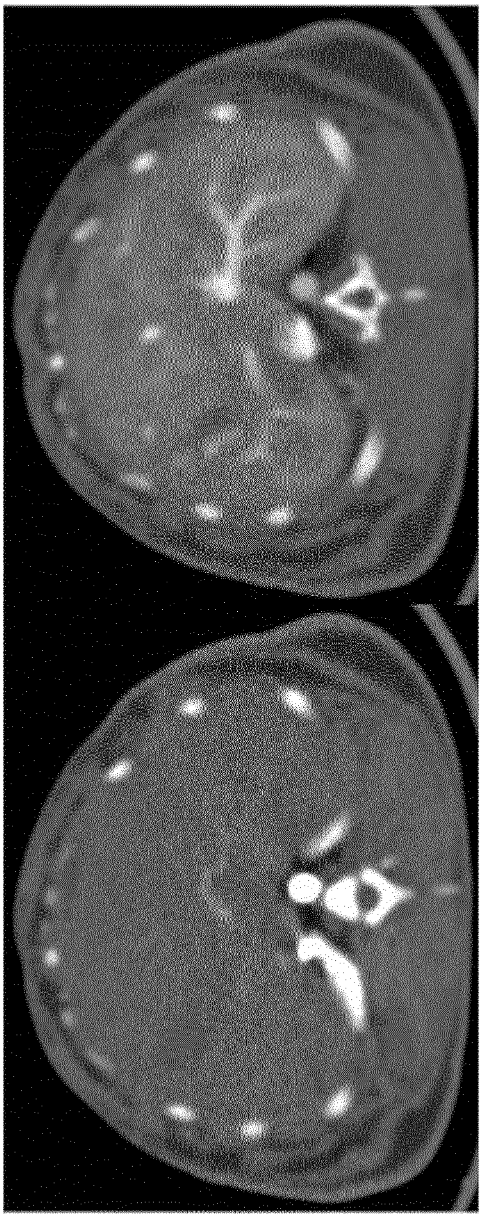

Fig. 2

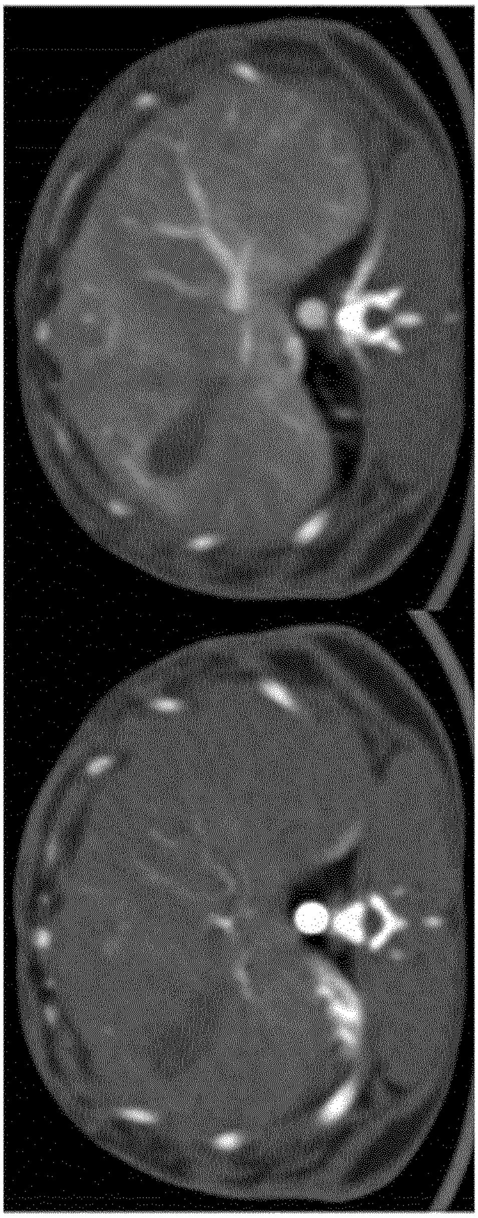

Fig. 3

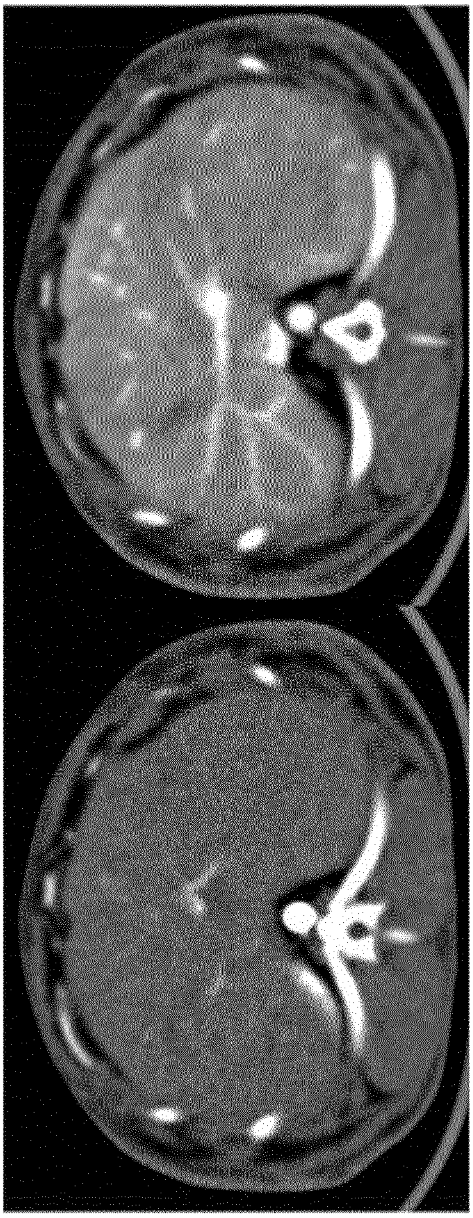

Fig. 4

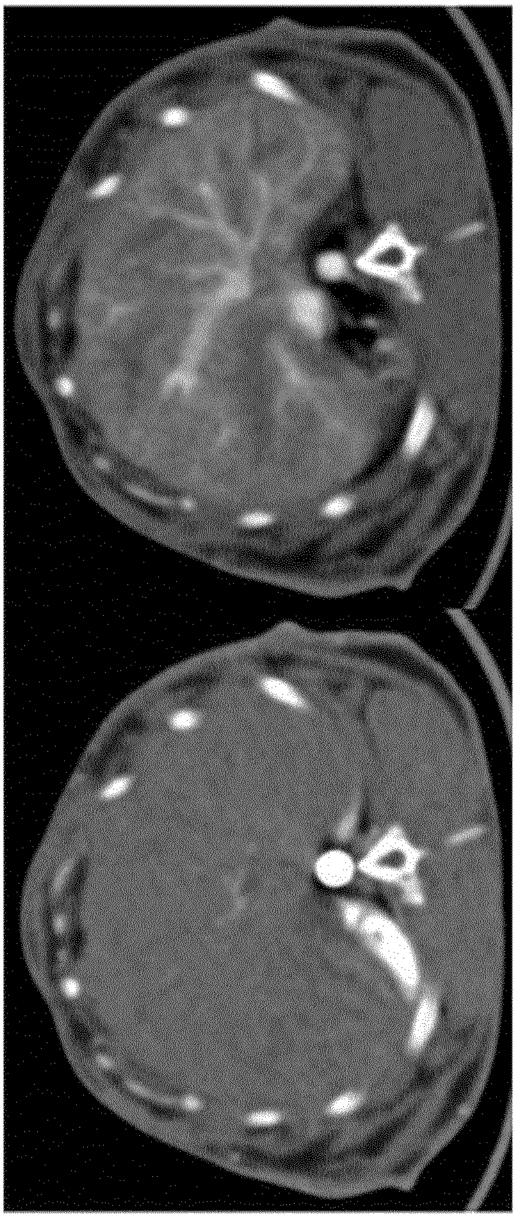

**Fig. 5**

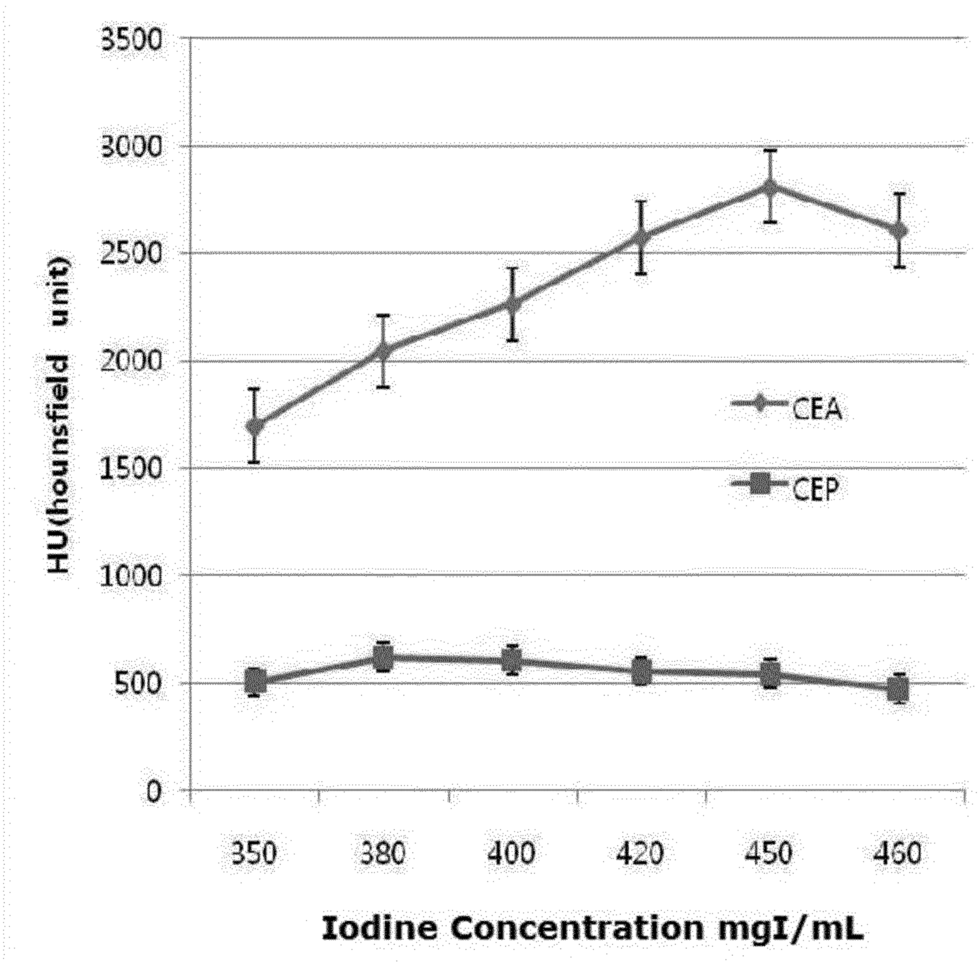
3500
3000
2500
2000
1500
1000
500
0
HU(hounsfield unit)
CEA
CEP
350
380
400
420
450
460
Iodine Concentration mgI/mL

Europäisches Patentamt
European Patent Office
Office européen des brevets

# EUROPEAN SEARCH REPORT

Application Number
EP 12 19 7686

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 98/23297 A2 (NYCOMED IMAGING AS [NO]; COCKBAIN JULIAN [GB]; OEDEGAARDSTUEN LIV INGR) 4 June 1998 (1998-06-04)<br>* page 4, line 28 - page 5, line 7 *<br>* page 10, line 15 - line 25 *<br>* page 13, line 27 - page 14, line 4 *<br>* examples 3, 7, 10, 14, 18 *<br>* page 4, line 9 - line 17 *<br>----- | 1-15 | INV.<br>A61K49/04 |
| X | WO 2007/116039 A1 (GUERBET SA [FR]; PETTA MYRIAM [FR]; LAVERGNE DAMIEN [FR]) 18 October 2007 (2007-10-18)<br>* page 3, line 15 - line 19 *<br>* page 5, line 25 - page 6, line 9 *<br>----- | 1,7-10 | |
| X | MALY, P.; FEX, G.: "CSF cation changes following subarachnoid injection of iohexol and metrizamide in rabbits", ACTA RADIOLOGICA: DIAGNOSIS, vol. 25, no. 1, 1984, pages 65-71, XP008160711,<br>* page 65, column 2, paragraph 3 - page 66, column 1, paragraph 1 *<br>* tables 1-2 *<br>* page 67, column 1, paragraph 2 *<br>* page 70, column 1, paragraph 2 *<br>----- | 1,2,7-10 | TECHNICAL FIELDS SEARCHED (IPC)<br>A61K |
| X | US 4 250 113 A (HOLTERMANN HUGO [NO] ET AL) 10 February 1981 (1981-02-10)<br>* column 4, line 7 - line 18 *<br>* column 4, line 30 - line 35 *<br>* examples 1-6 *<br>-----<br>-/-- | 1-3,7,9,10 | |

1

The present search report has been drawn up for all claims

| Place of search | Date of completion of the search | Examiner |
|---|---|---|
| The Hague | 19 March 2013 | Monami, Amélie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 19 7686

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| X | SI-JIA G ET AL: "The clinical application studies of CT spinal angiography with 64-detector row spiral CT in diagnosing spinal vascular malformations", EUROPEAN JOURNAL OF RADIOLOGY, ELSEVIER SCIENCE, NL, vol. 71, no. 1, 1 July 2009 (2009-07-01), pages 22-28, XP026223837, ISSN: 0720-048X, DOI: 10.1016/J.EJRAD.2008.04.005 [retrieved on 2008-05-21] * abstract * ----- | 1,2 | |
| X | RICHARD J OWEN ET AL: "Portal Vein Embolization with Radiolabeled Polyvinyl Alcohol Particles in a Swine Model: Hepatic Distribution and Implications for Pancreatic Islet Cell Transplantation", CARDIOVASCULAR AND INTERVENTIONAL RADIOLOGY, SPRINGER-VERLAG, NE, vol. 32, no. 3, 28 March 2009 (2009-03-28), pages 499-507, XP019707743, ISSN: 1432-086X * page 500, column 2, paragraph 2 * ----- | 1 | TECHNICAL FIELDS SEARCHED (IPC) |
| X | SOVAK M ET AL: "EVALUATION OF INTRATHECAL CONTRAST MEDIA BY AVERSION CONDITIONING IN RATS", INVESTIGATIVE RADIOLOGY, PHILADELPHIA, PA, US, vol. 17, no. 1, 1 January 1982 (1982-01-01), pages 101-106, XP008062183, * abstract * * page 103, column 1, paragraph 4 * ----- -/-- | 1 | |

The present search report has been drawn up for all claims

| Place of search | Date of completion of the search | Examiner |
|---|---|---|
| The Hague | 19 March 2013 | Monami, Amélie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

1

EPO FORM 1503 03.82 (P04C01)

Europäisches Patentamt
European Patent Office
Office européen des brevets

# EUROPEAN SEARCH REPORT

Application Number
EP 12 19 7686

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | R L Bree ET AL: "Cost-effective use of low-osmolality contrast media for CT of the liver: evaluation of liver enhancement provided by various doses of iohexol.", American Journal of Roentgenology, 1 January 1994 (1994-01-01), pages 579-583, XP055056111, DOI: 10.2214/ajr.163.3.8079849 Retrieved from the Internet: URL:http://www.ajronline.org/doi/pdf/10.2214/ajr.163.3.8079849 [retrieved on 2013-03-12] * abstract * * page 583, column 1, paragraph 4 - column 2, paragraph 1 * ----- | 1-15 | |

TECHNICAL FIELDS SEARCHED (IPC)

The present search report has been drawn up for all claims

1

| Place of search | Date of completion of the search | Examiner |
|---|---|---|
| The Hague | 19 March 2013 | Monami, Amélie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 12 19 7686

This annex lists the patent family members relating to the patent documents cited in the above-mentioned European search report.
The members are as contained in the European Patent Office EDP file on
The European Patent Office is in no way liable for these particulars which are merely given for the purpose of information.

19-03-2013

| Patent document cited in search report | Publication date | Patent family member(s) | Publication date |
|---|---|---|---|
| WO 9823297 A2 | 04-06-1998 | AU 5129398 A | 22-06-1998 |
| | | EP 1009445 A2 | 21-06-2000 |
| | | JP 2001504837 A | 10-04-2001 |
| | | WO 9823297 A2 | 04-06-1998 |
| WO 2007116039 A1 | 18-10-2007 | EP 2007712 A1 | 31-12-2008 |
| | | FR 2899581 A1 | 12-10-2007 |
| | | JP 2009532441 A | 10-09-2009 |
| | | US 2009143471 A1 | 04-06-2009 |
| | | WO 2007116039 A1 | 18-10-2007 |
| US 4250113 A | 10-02-1981 | BE 855580 A1 | 12-12-1977 |
| | | CH 630064 A5 | 28-05-1982 |
| | | DE 2726196 A1 | 22-12-1977 |
| | | DK 258477 A | 12-12-1977 |
| | | FR 2354316 A1 | 06-01-1978 |
| | | GB 1548594 A | 18-07-1979 |
| | | HK 56683 A | 25-11-1983 |
| | | JP S5321137 A | 27-02-1978 |
| | | JP S5654310 B2 | 24-12-1981 |
| | | NL 7706385 A | 13-12-1977 |
| | | SE 441181 B | 16-09-1985 |
| | | SE 7706792 A | 12-12-1977 |
| | | US 4250113 A | 10-02-1981 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- U. S. Pharmacopoeia. U. S. Pharmacopeial Convention, Rockville, MD. 1985, 1102
- Merck Index. Merck Co. Inc, 1989